# EUROPEAN PATENT SPECIFICATION

(11) **EP 3 810 078 B1**
(45) Date of publication and mention of the grant of the patent: **11.12.2024**
(21) Application number: 18923362.0
(22) Date of filing: 22.06.2018
(51) Int. Cl.: A61K 8/33, A61K 8/34, A61K 8/35, A61K 8/42, A61K 8/49, A61Q 11/00

(54) **DENTIFRICE COMPOSITIONS**
ZAHNPASTAZUSAMMENSETZUNGEN
COMPOSITIONS DE DENTIFRICE

(43) Date of publication of application: 28.04.2021
(73) Proprietor: The Procter & Gamble Company, Cincinnati, OH 45202 (US)
(72) Inventor: YUAN, Jinfang, Beijing 101312 (CN); LI, Xiaowei, Beijing 101312 (CN); SHANMUGAM, Thanigaivel, Beijing 101312 (CN)
(74) Representative: P&G Patent Germany
(86) International application number: PCT/CN2018/092340
(87) International publication number: WO 2019/241990

(56) References cited:
- WO-A1-2018/099570
- CN-A- 1 711 075
- CN-A- 101 238 203
- CN-A- 107 184 440
- CN-A- 107 184 440
- CN-A- 107 532 063
- JP-A- 2000 026 260
- US-A1- 2007 148 103
- US-A1- 2015 275 132

## Description

### FIELD OF THE INVENTION

The present invention relates to dentifrice compositions providing a dominant warming sensation in the oral cavity during toothbrushing and a subsequent dominant cooling sensation in the oral cavity after expelling the composition.

### BACKGROUND OF THE INVENTION

Dentifrice compositions are well known for dental and oral hygiene care. The use of peppermint essential oil comprising menthol or its derivatives to provide a cooling sensation is also well known (CN107184440A). Transient Receptor Potential Vanilloid-1 ("TRPV1") agonists are reported to provide a warming sensation, for example, when applied topically to skin. The combination of TRPV1 and menthol are generally described in personal care compositions. (US 2013/0315843 A1, US2015/275132A1, CN107532063A). WO2018/099570A1 discloses that adding hydroxyl compounds, in particular 1,2-alkandiol to TRPV1 and/or TRPV3 modulators, particularly to vanillyl ethers, simultaneously increases warming sensation of human skin or mucous membrane, while unwanted side effects of the modulators, such as skin irritation is reduced. However, a need is identified to provide a user sensorial experience when brushing teeth with a dentifrice where there is an initial dominant warming sensation experienced by the user within the two minutes of recommending tooth brushing time to signal the user the composition is working (e.g., to deliver an oral care benefit); but shortly thereafter (i.e., after brushing and expelling the dentifrice) a dominant cooling sensation is realized by the user so as to provide desired freshness experience that users have to come to expect from a dentifrice (e.g., for fresh breath, refreshing mouth feel, etc.). There is an opportunity to provide the user with such a sensorial experience from a dentifrice composition.

### SUMMARY OF THE INVENTION

The present invention is based, in part, on the surprising observation that a specific optimized ratio between a TRPV1 agonist and a menthol surprisingly provides such a user experience. Accordingly, one aspect of the invention provides a dentifrice composition comprising:
(a) a Transient Receptor Potential Vanilloid-1 ("TRPV 1") agonist;
(b) menthol, or a menthol derivative, or combination thereof, wherein the menthol derivative comprises the cyclohexane moiety, and is derivatized with functional groups including carboxamide, ketal, ester, ether, alcohol; and wherein the weight ratio of aforementioned (a):(b) is from 1:60 to 11:60, respectively
and wherein the composition further comprises a C6 alkanyl diol; a C8 alkanyl diol, or combination thereof.

Another aspect of the invention provides the dentifrice composition as disclosed herein for use inproviding:
(i) a dominant heating sensation to an oral cavity comprising the step brushing with a dentifrice composition described herein from 60 to 120 seconds; and
(ii) (ii) a subsequently dominant cooling sensation to the oral cavity after expelling said dentifrice composition from the brushed oral cavity, preferably for at least two minutes, more preferably three minutes.

One advantage of the present invention is to provide users a dominant warming sensation during brushing. Without wishing to be bound by theory, those users who experience a dominant warming sensation are given a reinforcing effect to signal the delivery of the benefit by the dentifrice composition (e.g., fluoride delivery etc.).

One advantage of the present invention is to provide users a dominant cooling sensation, i.e., freshness shortly after brushing. Without wishing to be bound by theory, those users who experience with the cooling sensation will obtain the freshness benefits that are well established and desired by users. In contrast, a dominant warming sensation after brushing may negatively impact a user's experience/desire for this cooling/freshening sensation. It may even send a mixed signal to users that there is too much of the benefit is being provided.

One advantage is the use of the dentifrice, and its aforementioned dominant warming and subsequent dominant cooling sensations, while minimizing irritation and/or undesirable astringency or metallic tastes.

One advantage is the cooling dominances starts shortly after expelling the dentifrice and rinsing (after brushing), i.e., within about 60 seconds, which is generally desirable by users.

Another advantage is the cooling dominance continues for at least two more minutes, even five or more minutes thereof, which is also generally desirable by users.

One advantage is the use of certain C6 or C8 diols to help mitigate against the lowering of pain threshold (in the oral cavity) when using TRPV1 agonist in the formulations described herein. This may help to mitigate against irritation or other negative effects.

While the specification concludes with claims that particularly point out and distinctly claim the invention, it is believed the present invention will be better understood from the following description.

### BRIEF DESCRIPTION OF THE DRAWINGS

While the specification concludes with claims particularly defining and distinctly claiming the invention, it is believed that the invention will be better understood from the following description of the accompanying figures. In the accompanying figures,
Figure 1 is the temporal dominance of sensation of inventive composition A;
Figure 2 is the temporal dominance of sensation of inventive composition B.

### DETAILED DESCRIPTION OF THE INVENTION

### Definitions

The term "effective amount" as used herein means an amount of a compound or composition sufficient to induce a positive benefit, an oral health benefit, and/or an amount low enough to avoid serious side effects, i.e., to provide a reasonable benefit to risk ratio, within the sound judgment of a skilled artisan. In one example, "effective amount" means at least 0.001% of the material, by weight of the composition, alternatively at least 0.1%.

The term "dentifrice" as used herein means paste, gel, powder, tablets, or liquid formulations, unless otherwise specified, that are used to clean the surfaces of the oral cavity. Preferably the dentifrice is a toothpaste. The term "teeth" as used herein refers to natural teeth as well as artificial teeth or dental prosthesis.

All percentages, parts and ratios are based upon the total weight of the compositions of the present invention, unless otherwise specified. All such weights as they pertain to listed ingredients are based on the active level and, therefore do not include solvents or by-products that may be included in commercially available materials, unless otherwise specified. The term "weight percent" may be denoted as "wt%" herein. All molecular weights as used herein are weight average molecular weights expressed as grams/mole, unless otherwise specified.

As used herein, the articles including "a" and "an" when used in a claim, are understood to mean one or more of what is claimed or described.

As used herein, the terms "comprise", "comprises", "comprising", "include", "includes", "including", "contain", "contains", and "containing" are meant to be non-limiting, i.e., other steps and other sections which do not affect the end of result can be added. The above terms encompass the terms "consisting of" and "consisting essentially of'.

As used herein, the words "preferred", "preferably" and variants refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

### TRPV1

The dentifrice composition comprises an effective amount of a Transient Receptor Potential Vanilloid-1 ("TRPV1") agonist; and also a C6 alkanyl diol and/or a C8 alkanyl diol, more preferably both the C6 alkanyl diol and the C8 alkanyl diol. Preferably the dentifrice composition comprises from 0.005% to 1%, preferably 0.01% to 0.8%, of the TRPV1 agonist by weight of dentifrice composition; alternatively from 0.01% to 0.2%, or from 0.03% to 0.12%, or from 0.04% to 0.11% by weight of the dentifrice composition. Non-limiting examples may include 0.01, 0.02, 0.03, 0.04, 0.05, 0.06, 0.07, 0.08, 0.09, 0.10, 0.11, or 0.12 wt% of the TRPV1 agonist by weight of the dentifrice composition. Alternatively, the dentifrice composition comprises less than 0.04%, or less than 0.035%, or less than 0.03% of the TRPV1 agonist by weight of the dentifrice composition, but greater than 0 wt%, or greater than 0.005 wt%.

The TRPV1 agonist is a capsaicin-responsive ligand-gated cation channel selectively expressed on small, unmyelinated peripheral nerve fibers (cutaneous nociceptors). Preferably the TRPV1 agonist is selected from the group consisting of: capsaicin, homocapsaicin, homodihydrocapsaicin, capsaicin derivatives, vanilloids (e.g., capsaicinoids), piperine, dialkdhyde sesquiterpene (e.g., warburganal, polygodial, isovelleral), triprenyl phenol (e.g., scutigeral), ginerols, shogaols, and combinations thereof. More preferably the TRPV1agonist is a vanilloid. Even more preferably the vanilloid is selected from the group consisting of N-vanillyl-alkanedienamides, N-vanillyl-alkanedienyls, N-vanillyl-cis-monounsaturated alkenamides, capsaicin, dihydrocapsaicin, norhydrocapsaicin, nordihydrocapsaicin, homocapsaicin, homodihydrocapsaicin, and combinations thereof. Yet still even more preferably the vanilloid is vanillyl butyl ether ("VBE"). Preferably the TRPV1 agonist is 10% to 70%, preferably 20% to 60%, more preferably 30% to 50%, by weight of the dentifrice composition. For example, the dentifrice composition may contain 35, 37, 39, 40, 41, 42, 44, 45 % of the TRPV1 agonist, by weight of the dentifrice composition. Preferably the TRPV1 agonist helps provide a perceivable warming sensation to the user without being too "hot" and minimizing any undesirable side effects such as burning, redness, or stinging. Other examples of TRPV1 agonists may include zingerone, or perhaps those identified in US Pat Publ. No. 2002/119231 A1.

### Diols

The dentifrice composition comprises a C6 alkanyl diol or a C8 alkanyl diol, preferably both the C6 and C8 alkanyl diols. Without wishing to be bound by theory, these diols may mitigate against the lowering of the pain threshold that may otherwise occur in using TRPV1 agonists without the diols. The practical application of this surprising discovery is the warming dominant effect without the negatives of increasing sensitivity. See the data in the Example below that illustrates this discovery. Preferably the C6 alkanyl diol is a C6 straight chain alkane diol. More preferably the C6 straight chain alkane diol is selected from 1,2-hexandiol, 1,6-hexandiol, mixture thereof. Yet more preferably the C6 straight chain alkane diol is 1,2-hexanediol. Preferably the C8 alkanyl diol is a C8 straight chain alkane diol. More preferably the C8 straight chain alkane diol is selected from 1,2-octanediol (also known as "caprylyl glycol"), 1,8-octanediol, mixture thereof. Yet more preferably the C8 straight chain alkane diol is 1,2-octanediol. More preferably the dentifrice composition comprises 1,2-hexanediol (as the C6 alkanyl diol) and 1,2-octanediol (as the C8 alkanyl diol). In one example, the dentifrice composition is substantially free (e.g., less than 0.001 wt% of the dentifrice composition), preferably free, of C10 diol; and preferably is substantially, preferably free, of a C5 and C4 diols. Without wishing to be bound by theory, the presence of C10 diol may contribute to a stringent or bitter or otherwise undesirable taste. The presence of C5 or C4 diol, without wishing to be bound by theory, may not provide the same desirable degree of mitigation against pain threshold lowering (as compared to said combination of C6 and C8 diols). The dentifrice composition may optionally comprise an antioxidant, such as ascorbyl palmitate. In an alternative example, the dentifrice composition is substantially free (e.g., less than 0.001 wt%), preferably free, of alkanyl diols other then said C6 alkanyl diol or said C8 alkanyl diol. In one example, the weight ratio of (TRPV1 agonist) : (said C6 alkanyl diol, said C8 alkanyl diol, or said combination thereof) is from 4:1 to 4:16, respectively; preferably from 4:3 to 4:9.

### Menthol or Menthol Derivative

The dentifrice composition comprises from 0.001% to 1% of a menthol, or menthol derivative, or combination thereof, by weight of the dentifrice composition. Preferably from 0.01 to 0.7 wt%, more preferably from 0.1 wt% to 0.6 wt%, yet more preferably greater than 0.2 wt% to 0. 4 wt%, of said menthol, menthol derivative, or combination thereof. Without wishing to be bound by theory, having too high a level of menthol or menthol derivative may interfere with a dominant warming sensation during brushing or may require too high of TRPV1 agonist which could cause irritation or some other undesirable effect with such relatively high levels of the TRPV1 agonist.

A menthol derivative is one that is structurally related to menthol, i.e., containing the cyclohexane moiety, and derivatized with functional group including carboxamide, ketal, ester, ether, alcohol, or combinations thereof. Preferably the menthol or menthol derivative is selected from one or more of the following: L-menthol ((1R,2S,SR)-5-methyl-2-propan-2-ylcyclohexane), 1-menthol ((2S,SR)-5-methyl-2-propan-2-ylcyclohexan- 1-one), or menthone (5-methyl-2-propan-2-ylcyclohexan-1-one); more preferably 1-menthol. Without wishing to be bound by theory, menthol provides a cooling sensation to users. As used herein, "menthol derivative" are chemical derivatives of or are structurally related to menthol, i.e., containing the cyclohexane moiety, and derivatized with functional groups including carboxamide, ketal, ester, ether and alcohol. Examples include the p-menthanecarboxamide compounds such as N-ethyl-p-menthan-3-carboxamide or N-(4-cyanomethylphenyl)-ρ-menthanecarboxamide (EVERCOOL^{®} 180).

The weight ratio between the TRPV1 agonist and menthol or menthol derivative is of critical importance. It is the weight ratio relationship between these two ingredients that provides the desired balance of warming dominance effect while brushing and a cooling dominance effect after rinsing. One aspect of the invention provides a weight ratio of 1:60 to 11:60 of these ingredients, respectively. Preferably the weight ratio is from 2:60 to 11:60, preferably from 3:60 to 11:60, more preferably from 5:60 to 10:60, yet more preferably from 7:60 to 9:60, alternatively combinations thereof. Non-limiting weight ratio examples include: 1:60, 2:60, 3:60, 4:60; 5:60; 6:60, 7:60; 8:60; 9:60; 10:60; and 11:60.

### Abrasive

The dentifrice composition comprises an effective amount of an abrasive. Examples of abrasives include a calcium-containing abrasive, a silica, or combination thereof. If containing a calcium-containing abrasive, the calcium-containing abrasive is preferably selected from the group consisting of calcium carbonate, dicalcium phosphate, tricalcium phosphate, calcium orthophosphate, calcium metaphosphate, calcium polyphosphate, calcium oxyapatite, sodium carbonate, sodium bicarbonate, and combinations thereof. If a silica, preferably the silica is a precipitated silica (e.g., sodium silicate solution by destabilizing with acid as to yield very fine particles) such as those from the ZEODENT^{®} series from Huber Engineered Materials (e.g., ZEODENT^{®} 103, 124, 113 115, 163, 165, 167). It is acknowledged that some of these silicas (e.g., synthetic amorphous silica) can perform both abrasive and thickening functions, but are included herein under the term "abrasive" for purposes of the present invention. Preferably the dentifrice composition comprises from 1% to 35%, more preferably from 5% to 25% of abrasive, by weight of the composition.

### Humectants

Optionally, but preferably, the dentifrice compositions comprise an effective amount of a humectant. The term "humectant," for the purposes of present invention, include edible polyhydric alcohols such as glycerin, sorbitol, xylitol, butylene glycol, propylene glycol, erythritol, maltol, mannitol and combinations thereof. In another example, the humectant is selected from sorbitol, glycerin, and combinations thereof. Preferably the humectant is sorbitol. In an example, the composition comprises from 0% to 66%, alternatively from 40% to 55 %, of humectant by weight of the dentifrice composition.

### Thickening Polymer

Optionally, but preferably, the dentifrice composition comprises an effect amount of a thickening polymer. Preferably the thickening polymer comprises from 0.1% to 10% by weight of the dentifrice composition. An example of a thickening polymer is a linear sulfated polysaccharide. In turn, carrageenans or carrageenins are one example of a linear sulfated polysaccharide. Generally, carrageenans can vary based upon the degree of sulfation that include: Kappa-carrageenan, Iota-carrageenan, and Lambda-carrageenan. Combinations of carrageenans can be used.

Another example of a thickening polymer is polyethylene glycol (PEG). PEG is available in a various weight percentages as well as various ranges of average molecular weights, for example, from 100 Daltons to 1600 Daltons. PEG is a water soluble linear polymer formed by the addition reaction of ethylene oxide to an ethylene glycol equivalent having the general formula is: H-(OCH₂CH₂)ₙ-OH.

Yet another example of a thickening polymer is a carboxymethyl cellulose ("CMC"). CMC is prepared from cellulose by treatment with alkali and monochloro-acetic acid or its sodium salt. Different varieties are commercially characterized by viscosity. One commercially available example is Aqualon^{™} branded CMC from Ashland Special Ingredients (e.g., Aqualon^{™} 7H3SF; 9M3SF; TM9A; TM12A).

The thickening polymer may include one or more of the aforementioned polymer types. Preferably the dentifrice composition comprises from 0.15 to 10% of the thickening polymer by weight of the dentifrice composition.

Preferably the dentifrice compositions of the present invention have a viscosity range from 20,000 10⁻³Pa·s (centipoise) to two million 10⁻³Pa·s (centipoise), preferably 100,000 10⁻³Pa·s (centipoise) to 850,000 10⁻³Pa·s (centipoise), more preferably from 150,000 10⁻³Pa·s (centipoise) to 600,000 10⁻³Pa·s (centipoise) ("cP"). This may include compositions such a gel with a low viscosity that is dispensed via a pump or a paste having a high viscosity that is manually squeezed from a tube. A method for assessing viscosity is described. The viscometer is Brookfield^{®} viscometer, Model DV-I Prime with a Brookfield "Helipath" stand. The viscometer is placed on the Helipath stand and leveled via spirit levels. The E spindle is attached, and the viscometer is set to 2.5 RPM. Detach the spindle, zero the viscometer and install the E spindle. Then, lower the spindle until the crosspiece is partially submerged in the paste before starting the measurement. Simultaneously turn on the power switch on the viscometer and the helipath to start rotation of the spindle downward. Set a timer for 48 seconds and turn the timer on at the same time as the motor and helipath. Take a reading after the 48 seconds. The temperature is at ambient conditions. The reading is in cP.

### Water

The dentifrice compositions of the present invention may comprise water. The water may be added to the formulation and/or may come into the composition from the inclusion of other ingredients. The dentifrice composition comprises from 5% to 75%, alternatively from 15% to 30% water, by weight of the dentifrice composition.

### Fluoride ion source

Optionally, but preferably, the dentifrice compositions may include an effective amount of a fluoride ion source. The fluoride ion may be present in an amount sufficient to give a fluoride ion concentration in the composition at 25° C, and/or in one embodiment can be used at levels of from about 0.0025% to about 5% by weight of the composition. Examples of fluoride ion sources include: stannous fluoride, sodium fluoride, potassium fluoride, amine fluoride, sodium monofluorophosphate, and zinc fluoride.

The method for assessing soluble fluoride is described consistent with the China's National Standard Method GB8372-2008. Briefly, an ion-selective electrode (ISE) is used to test soluble fluoride in dentifrice. An example of a fluoride ion meter is SARTORIUS PP-50, but an equivalent may be used.

### pH

The pH of the dentifrice composition is preferably from 4.5 to 10, preferably from 6 to 8, alternatively from 6.5 to 7.5. The pH of the composition may be adjusted with a strong acid (e.g., hydrochloric acid) or a strong base (e.g., sodium hydroxide). A method for assessing pH of dentifrice is described. pH is measured by a pH Meter with Automatic Temperature Compensating (ATC) probe. The pH Meter is capable of reading to 0.001 pH unit. The pH electrode may be selected from one of the following (i) Orion Ross Sure-Flow combination: Glass body - VWR #34104-834/Orion #8172BN or VWR#10010-772/Orion #8172BNWP; Epoxy body - VWR #34104-830/Orion #8165BN or VWR#10010-770/Orion #8165BNWP; Semi-micro, epoxy body - VWR #34104-837/Orion #8175BN or VWR#10010-774/Orion #3175BNWP; or (ii) Orion PerpHect combination:VWR #34104-843/Orion #8203BN semi-micro, glass body; or (iii) suitable equivalent. The automatic temperature compensating probe is Fisher Scientific, Cat #13-620-16.

A 25% by weight slurry of dentifrice is prepared with deionized water, and thereafter is centrifuged for 10 minutes at 15000 rotations-per-minute using a SORVALL RC 28S centrifuge and SS-34 rotor (or equivalent gravitational force, at 24149g force). The pH is assessed in supernatant after one minute or the taking reading is stabilized. After each pH assessment, the electrode is washed with deionized water. Any excess water is wiped with a laboratory grade tissue. When not in issue, the electrode is kept immersed in a pH 7 buffer solution or an appropriate electrode storage solution.

### Surfactant

Optionally, but preferably, the dentifrice compositions comprise a surfactant. The surfactant may be selected from anionic, nonionic, amphoteric, zwitterionic, cationic surfactants, or combination thereof, preferably the surfactant is anionic, more preferably the anionic surfactant is sodium lauryl sulfate (SLS). An example of a zwitterionic surfactant is cocamidopropyl betaine. The dentifrice composition may contain one, two, or more surfactants. The composition may include a surfactant at a level of from 0.1% to 10%, by weight of the total composition.

### EXAMPLES

Comparative and inventive dentifrice compositions, specifically toothpaste, are made according to the ingredient list in Table 1. Table 2 provides the results of testing the dentifrice compositions before an expert sensory panel where the dominant sensation (i.e., warming or cooling) is assessed before and after brushing with these compositions.

Turning to Table 1, the material difference between the comparative and inventive composition is the weight ratio of the TRPV1 agonist, i.e., VBE to that of the menthol. Specifically, the inventive compositions has a higher weight ratio of menthol to the TRPV1 agonist relative to the comparative example. The amount of VBE (0.04 wt%) is constant between the composition, but the inventive composition has slightly more 1-menthol (at 0.3 wt% for inventive vs. 0.2 wt% for comparative).

**Table 1: Ingredients and Weight Percentages (Wt%) of comparative and Inventive Dentifrice Examples are Provided**

| Ingredient: | Weight Percentage (Wt%) | | |
|---|---|---|---|
| | Comparative | Inventive A | Inventive B |
| 1-Menthol | 0. 2 | 0.3 | 0.24 |
| Sensating Composition¹ | 0.1 (0.04 of VBE) | 0.1 (0.04 of VBE) | 0.08 (0.032 of VBE) |
| Weight ratio of VBE: 1-menthol | 12:60 | 8:60 | 8:60 |
| Sorbitol solution (70 wt% active) | 66 | 66 | 66 |
| Water added (treated) | q.s | q. s | q. s |
| Sodium fluoride | 0.243 | 0.243 | 0.243 |
| monobasic sodium phosphate | 0.4 | 0.4 | 0.4 |
| Sodium phosphate tribasic dodecahydrate | 0.93 | 0.93 | 0.93 |
| Saccharin sodium | 0.25 | 0.25 | 0.25 |
| Carboxymethyl cellulose | 0.7 | 0.7 | 0.7 |
| Carbomer | 0.22 | 0.22 | 0.22 |
| Silica (ZEODENT^{®}) | 16 | 16 | 16 |
| Sodium lauryl sulfate (29 wt% active) | 6.75 | 6.75 | 6.75 |
| Flavor - Green Tea | 0.9 | 0.9 | 0.9 |
| | | | |
| Target Viscosity (BKU²) | 8-37 | 8-37 | 8-37 |
| Target pH | 6.5-7.5 | 6.5-7.5 | 6.5-7.5 |

| | | | |
|---|---|---|---|
| ¹ "Sensating Composition" is THERMOLAT^{®} from Symrise, product number 793238. The commercial literature from Symrise (dated 2017) reports 1 wt% of THERMOLAT^{®} has 0.4% vanillyl butyl ether (i.e., "VBE" as the TRPV1 agonist) wherein the remaining amount has unspecified levels of the diols 1,2-hexandiol, caprylyl glycol (i.e., 1,2-octanediol), and antioxidant ascorbyl palmitate. ²1 BKU = 10,000 10⁻³Pa·s (centipoise) | | | |

Table 2 provides the results of a 12-member sensory expert panelists assessing the dominant warming or cooling sensation experience at different time points during and after brushing teeth with the comparative and inventive compositions. The comparative and inventive composition are those described in Table 1 above. The expert panelists are trained in assessing flavors and sensates, especially in dentifrice compositions such as toothpaste. For this study, panelists are asked whether there is a dominant warming or cooling sensation at the indicated time points. Time point zero begins when the panelist begins brushing their teeth (with the subject dentifrice composition). The panelist brushes for two minutes before immediately expelling the dentifrice composition. The oral cavity is then immediately rinsed for a few seconds with room temperature tap water and expelled. The number of panelists that perceived either a dominant warming sensation or dominant cooling sensation are reported at time points 30, 60, 90, 120 seconds (during brushing): and at 180, 240, and 300 seconds (after brushing). If the panelist could not determine whether a warm or cooling sensation was dominant at the designated time point, the result is not tabulated in either the warming or cooling column. Results are provided in Table 2 below.

**Table 2A: Assessing a dominating warming or cool sensation at different time points during and after toothbrushing with comparative and inventive toothpaste composition A.**

| | Time (Sec) | Comparative Comp. | | P-value * = significant | Inventive Comp. A | | P-value * = significant |
|---|---|---|---|---|---|---|---|
| | | Warming | Cooling | | Warming | Cooling | |
| During Brushing | 30 | 10 | 2 | 0.045 * | 6 | 6 | 1 |
| | 60 | 9 | 2 | 0.066 * | 9 | 3 | 0.118 |
| | 90 | 11 | 1 | 0.016 * | 9 | 2 | 0.066 * |
| | 120 | 9 | 2 | 0.066 * | 7 | 5 | 0.583 |
| After Brushing | 180 | 5 | 7 | 0.583 | 2 | 9 | 0.066 * |
| | 240 | 4 | 8 | 0.282 | 1 | 10 | 0.024 * |
| | 300 | 3 | 9 | 0.118 | 1 | 7 | 0.079 * |

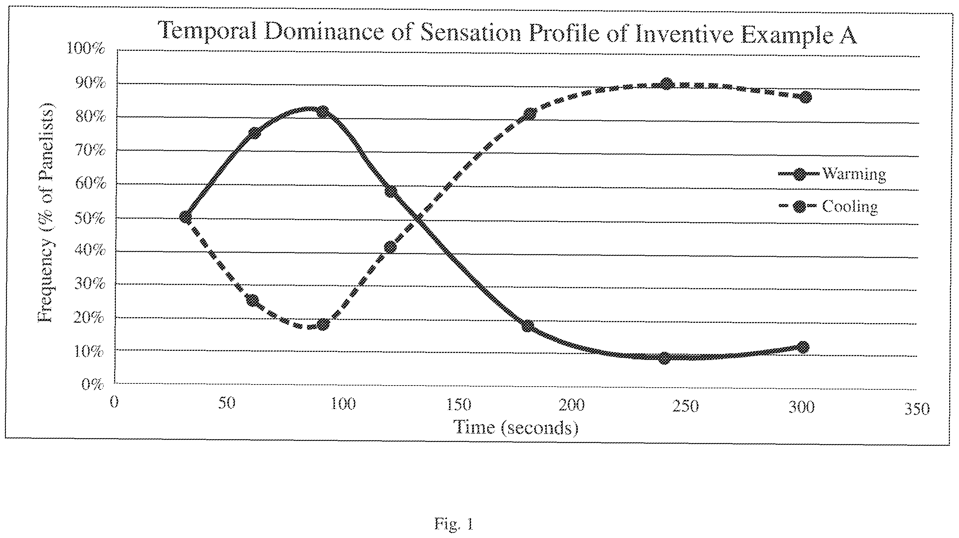

As provided in Table 2A, and illustrated in Figure 1, the inventive dentifrice composition A provides both a dominant warming sensation during brushing and a dominant cooling sensation after brushing. This is a desirable experience by user who are reinforced a message of active or benefit delivery to the oral cavity during brushing but also are provided a dominant cooling sensation after brushing. The single variable between the comparative and inventive compositions is the amount of menthol. That is, the inventive composition has a higher amount of menthol, i.e., a higher weight ratio between the menthol and TRPV1 agonist to provide the balance between the desired dominant cooling sensation, and at all of the 180, 240, and 300 second time points. The results are surprising given the relatively small difference in the weight ratio relationship between these ingredients provides such a dramatic difference in results.

**Table 2B: Assessing a dominating warming or cool sensation at different time points during and after toothbrushing with inventive toothpaste composition B.**

| | Time (Sec) | Inventive Composition B | | Significance @ 90% confidence level |
|---|---|---|---|---|
| | | Warming | Cooling | |
| During Brushing | 30 | 9 | 2 | Yes |
| | 60 | 10 | 1 | Yes |
| | 90 | 9 | 1 | Yes |
| | 120 | 9 | 2 | Yes |
| After Brushing | 180 | 5 | 6 | No |
| | 240 | 2 | 9 | Yes |
| | 300 | 2 | 9 | Yes |

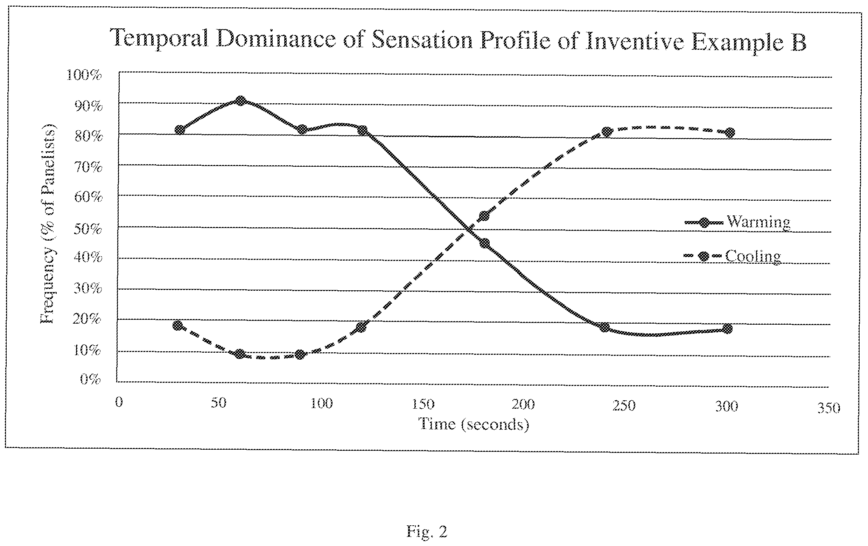

As provided in Table 2B, and illustrated in Figure 2, the inventive dentifrice composition B provides both a dominant warming sensation during brushing and a dominant cooling sensation after brushing. Eleven sensory expert panelists are used in generating the data of Table 2B. The weight ratio of VBE: 1-menthol is the same between inventive compositions A and B (i.e., 8:60, respectively); however inventive composition B contains 80% less of the amount of VBE and 1-menthol. Nevertheless, comparable results are observed between the inventive compositions thereby indicating the importance of the weight ratio in achieving the aforementioned benefits.

Data is provided to demonstrate the impact of having diols in the dentifrice composition to help mitigate against lowering of the pain threshold in the oral cavity that otherwise happens in the presence of VBE (i.e., a TRPV1 agonist). An electrical current perception threshold (CPT) evaluating device is used to assess the trigeminal nerve at 5 Hertz (Hz) and 250 Hz in compositions with and without diols. Results are reported as milliamps. Using microprocessor controlled, neuroselective electrical stimuli, the instrument quickly quantifies the conduction and functional integrity of the large and small myelinated and small unmyelinated sensory nerve fibers at any cutaneous site. Alternatively, the mucosal or gum area may also be assessed (but not assessed in this study). The device used is the a Neurometer^{®} CPT^{®}/C neuroselective sensory Nerve Conduction Threshold (sNCT^{™}) electrodiagnostic testing device, from Neurotron, Incorporated (Denver, CO, USA). See Operating Manual, Version18.3, copyright 2010; and www.neutron.com.

Electrical CPT evaluation quantifies the sensory threshold to transcutaneous electrical stimulation of the sensory nerves. The data herein shows a significant correlation between CPT and skin sensation. It is known that sensations (e.g., cooling, heat, pressure, pain, tingling, and the like) from mouth are transmitted into brain through the trigeminal nerve. Therefore, CPT measurement at, for example, the mandibular division of this nerve is a way to objectively understand how users of oral care compositions respond to different sensates.

Two groups of 10-15 subjects in each group are provided. Subjects are randomized for gender and age. The test is conducted, in the relevant portion, over five days. Subjects are preconditioned before the test. Members of the first group brush their teeth with Example C toothpaste (in Table 3 below) and the second group with Example D toothpaste. The notable difference between these two compositions is the presence of diols. That is, example D contains diols whereas example C does not. Although both compositions have 0.45 wt% of menthol, the point of this study is to measure how diols may reduce pain, i.e., minimize the reduction of the pain threshold (vs. e.g., assessing the sensorial experience).

**Table 3: Ingredients and Weight Percentages (Wt%) of Toothpaste Compositions Examples C and D are provided.**

| Ingredient: | Example C (Wt%) | Example D (Wt%) |
|---|---|---|
| 1-Menthol | 0.45 | 0.45 |
| TRPV1 agonist | 0.04 of VBE (nil diols) | 0.1 of THERMOLAT^{®3} (0.04 of VBE) |
| Sorbitol solution (70 wt% active) | 66 | 66 |
| Water added (treated) | q. s | q. s |
| Sodium fluoride | 0.243 | 0.243 |
| monobasic sodium phosphate | 0.4 | 0.4 |
| Sodium phosphate tribasic dodecahydrate | 0.93 | 0.93 |
| Saccharin sodium | 0.30 | 0.30 |
| Carboxymethyl cellulose | 0.7 | 0.7 |
| Carbomer | 0.22 | 0.22 |
| Silica (ZEODENT^{®}) | 16 | 16 |
| Sodium lauryl sulfate (29 wt% active) | 7.0 | 7.0 |
| Flavor - citrus mint (excluding menthol) | 0.55 | 0.55 |
| | | |
| Target Viscosity (BKU⁴) | 8-37 | 8-37 |
| Target pH | 6.5-7.5 | 6.5-7.5 |

| | | |
|---|---|---|
| ³ THERMOLAT^{®} is from Symrise, product number 793238. The commercial literature from Symrise (dated 2017) reports 1 wt% of THERMOLAT^{®} has 0.4% vanillyl butyl ether (i.e., "VBE" as the TRPV1 agonist) wherein the remaining amount has unspecified levels of the diols 1,2-hexandiol, caprylyl glycol (i.e., 1,2-octanediol), and antioxidant ascorbyl palmitate. ⁴1 BKU = 10,000 10⁻³Pa·s (centipoise) | | |

The following steps are taken for each of the five days of the study. The CPT assessment is undertaken at Day 1 and then again at Day 5. Specifically, the assessment is taken before brushing and after brushing on the first day. Lastly, the assessment is taken at the fifth day. At least one hour before each assessment, there is no eating, drinking, chewing gum, or toothbrushing (so as not to interfere with the results). The treatment or test for each day is conducted in a clinical laboratory.

**Table 4: Summary of the CPT test method over five days.**

| Day: | Treatment or Test: |
|---|---|
| 1 | Step 1: CPT test at the mandibular division of the trigeminal nerve ("Jaw"). |
| | Step 2: Brush teeth with composition C or D. |
| | Step 3: CPT test at Jaw. |
| 2 | Use subject toothpaste. |
| 3 | Use subject toothpaste. |
| 4 | Use subject toothpaste. |
| 5 | CPT test at Jaw. |

Results for the two groups (using toothpaste example C or D, respectively) are provided in Table 5A. Results provided at the 5 Hz and 250 Hz as well as an averaging of the two frequencies. A P value, to determine whether there are any statistically significant differences, are provided in Table 5B below.

**Table 5A: Least Square means at 5 Hz / 250 Hz / 5 + 250 Hz**

| Ex.: | Hz | Least Square Means (Standard Error) | | |
|---|---|---|---|---|
| | | Day 1 Before Brushing | Day 1 After Brushing | Day 5 Before Brushing |
| C | 5 | 14.5 (2.4) | 7.7 (1.7) | 13.7 (1.8) |
| D | 5 | 12.9 (2.4) | 12.2 (1.7) | 14.7 (1.8) |
| C | 250 | 26.1 (3.5) | 17.6 (2.9) | 30.1 (3.3) |
| D | 250 | 26.7 (3.5) | 26.4 (2.9) | 30.2 (3.3) |
| C | 5+250 | 39.8 (5.5) | 25.1 (4.2) | 43.4 (4.6) |
| D | 5+250 | 39.8 (5.5) | 38.3 (4.2) | 45.3 (4.6) |

**Table 5B: P values with respect to Table 5A**

| Ex.: | Hz | P value | | |
|---|---|---|---|---|
| | | Day 1 Before Brushing vs. Day 1-After Brushing | Day 1 Before Brushing vs. Day 5 Before Brushing | Day 1 After Brushing vs. Day 5 Before Brushing |
| C | 5 | 0.0004 (*Significant*) | 0.7569 | 0.0245 (*Significant*) |
| D | 5 | 0.6267 | 0.4841 | 0.3131 |
| C | 250 | 0.003 (*Significant*) | 0.4144 | 0.0037 (*Significant*) |
| D | 250 | 0.8885 | 0.4852 | 0.3337 |
| C | 5+250 | 0.0006 (*Significant*) | 0.6131 | 0.0054 (*Significant*) |
| D | 5+250 | 0.6856 | 0.4427 | 0.2529 |

Results of Table 5A and 5B are discussed. An acute response of the diols is provided in Day 1 results. A cumulative response is provided by the diols when comparing between Day 1 and Day 5 results at 5 Hz. There is a statistically significant difference discovered on Day 1, before and after brushing, when subjects use toothpaste composition C (which does not contain diols). In contrast, there is no difference assessed on Day 1 for those users using toothpaste composition D (which contains diols). The difference between these compositions is the presence of diols. The presence of diols in Example D suggests the pain threshold does not decrease in the presence of diols in contrast to when the diols are not present (and the pain threshold decreases). Without wishing to be bound by theory, this decrease in the pain threshold may make the user more sensitive to irritants in the oral cavity.

A cumulative response of the diols is provided when comparing Day 1 after brushing and Day 5 before brushing. That is, there is a statistically significant difference discovered when subjects use toothpaste C at this time-point, in contrast to comparing to those subjects using toothpaste D. Net, the presence of diols in example D suggests the pain threshold does not decrease over this time-period as compared to when the diols are not present (and the pain threshold decreases).

Analogous results are observed when the study is conducted at 250 Hz and when averaging the results of 5 Hz and 250 Hz. Thus, the presence of diols can help provide dentifrice compositions, of the present invention, which not only provides the desired dominant heating and subsequent dominant cooling benefits, but also provides the user the additional benefit of helping to keep the pain threshold from decreasing (thereby helping the user not become more susceptible to irritants in the oral cavity).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A dentifrice composition comprising:
(a) a Transient Receptor Potential Vanilloid-1 ("TRPV1") agonist;
(b) menthol, or a menthol derivative, or combination thereof, wherein the menthol derivative comprises the cyclohexane moiety, and is derivatized with functional groups including carboxamide, ketal, ester, ether, alcohol; and
wherein the weight ratio of aforementioned (a):(b) is from 1:60 to 11:60, respectively; and
wherein the composition further comprises a C6 alkanyl diol; a C8 alkanyl diol, or combination thereof.

2. The composition of claim 1, wherein said weight ratio of said (a):(b) is from 2:60 to 11:60, respectively; preferably from 5:60 to 10:60; more preferably 7:60 to 9:60.

3. The composition of claim 1 or 2, wherein the composition comprises the combination of a C6 alkanyl diol and a C8 alkanyl diol.

4. The composition of any of the preceding claims, wherein the weight ratio of (TRPV1 agonist) : (said C6 alkanyl diol, said C8 alkanyl diol, or said combination thereof) is from 4:1 to 4:16, respectively; preferably from 4:3 to 4:9.

5. The composition of any of the preceding claims, wherein the composition comprises from 0.005% to 1% of the TRPV1 agonist, by weight of the composition; preferably from 0.01 to 0.08 wt%, more preferably from 0.02 to 0.06 wt%, yet still more preferably from 0.03 to 0.05 wt% of said TRPV1 agonist, by weight of the dentifrice composition.

6. The composition of any of the preceding claims, wherein the composition comprises from 0.001% to 1 % of the menthol, menthol derivative, or combination thereof, by weight of the composition; preferably from 0.01 to 0.7 wt%, more preferably from 0.1 wt% to 0.6 wt%, yet more preferably greater than 0.2 wt% to 0. 4 wt%, of said menthol, menthol derivative, or combination thereof.

7. The dentifrice composition of any one of the preceding claims, wherein the menthol or menthol derivative is selected from L-menthol, 1-menthol, methone, and combinations thereof; preferably 1-menthol.

8. The dentifrice composition of any one of the preceding claims, wherein the C6 alkanyl diol is a C6 straight chain alkane diol;
preferably the C6 straight chain alkane diol is selected from 1,2-hexandiol, 1,6-hexandiol, mixture thereof;
more preferably the C6 straight chain alkane diol is 1,2-hexanediol.

9. The dentifrice composition of any one of the preceding claims, wherein the C8 alkanyl diol is a C8 straight chain alkane diol;
preferably the C8 straight chain alkane diol is selected from 1,2-octanediol, 1,8-octanediol, mixture thereof;
more preferably the C8 straight chain alkane diol is 1,2-octanediol.

10. The dentifrice composition of any one of the preceding claims, wherein the C6 alkanyl diol and C8 alkanyl diol are 1,2-hexanediol and 1,2-octanediol, respectively.

11. The dentifrice composition of any one of the preceding claims, wherein the TRPV1 agonist is selected from the group consisting of: capsaicin, vanilloids, piperine, dialkdhyde sesquiterpene, triprenyl phenol, ginerols, shogaols, and combinations thereof;
preferably wherein the TRPV1 agonist is a vanilloid;
more preferably the vanilloid is selected from N-vanillyl-alkanedienamides, N-vanillyl-alkanedienyls, N-vanillyl-cis-monounsaturated alkenamides, capsaicin, dihydrocapsaicin, norhydrocapsaicin, nordihydrocapsaicin, homocapsaicin, homodihydrocapsaicin, and combinations thereof;
yet more preferably the vanilloid is vanillyl butyl ether.

12. The dentifrice composition of any one of the preceding claims, further comprising from 0.1% to 25% of an abrasive, by weight of the composition; preferably wherein the abrasive is silica, more preferably the composition comprises from 5 to 25 wt% of the silica.

13. The dentifrice composition of any one of the preceding claims, further comprising a humectant;
preferably the humectant is a polyol;
more preferably the polyol is selected from sorbitol, glycerin, or combination thereof;
even more preferably from 1% to 60% of the humectant, preferably from 40% to 55%, by weight of the dentifrice composition.

14. The dentifrice composition of any one of the preceding claims comprising:
(a) water, preferably 5% to 30% of water, more preferably from 15% to 25% of water, by weight of the dentifrice composition;
(b) a thickening polymer, preferably wherein the thickening polymer comprises from 0.1% to 10% by weight of the dentifrice composition;
(c) a pH from 5 to 9, preferably from 6 to 8, more preferably from 6.5 to 7.5; and
(d) a viscosity from 150,000 10⁻³Pa·s (centipoise) to 850,000 10⁻³Pa·s (centipoise) measured at ambient temperature conditions with a Brookfield ^{®} viscometer, Model DV-I Prime with a Brookfield "Helipath" stand, wherein the viscometer is placed on the Helipath stand and leveled via spirit levels; the E spindle is attached, and the viscometer is set to 2.5 rpm; then the spindle is detached, the viscometer is zeroed and the E spindle is installed and lowered until the crosspiece is partially submerged in the paste before starting the measurement; the power switch on the viscometer and the helipath is turned on simultaneously to start rotation of the spindle downward and the viscosity is read after 48 seconds in centipoise at ambient temperature; and
(e) optionally said dentifrice composition comprises less than 0.001wt% of, preferably is free, of alkanyl diols other then said C6 alkanyl diol or said C8 alkanyl diol.

15. Dentifrice composition of claims 1-14 for use in providing:
(i) a dominant heating sensation to an oral cavity comprising the step brushing with the dentifrice composition from 60 seconds to 120 seconds; and
(ii) a subsequently dominant cooling sensation to the oral cavity after expelling said dentifrice composition from the brushed oral cavity.

## Patentansprüche

1. Zahncremezusammensetzung, umfassend:
(a) einen Transient-Receptor-Potential-Vanilloid-1-Agonisten ("TRPV1");
(b) Menthol oder ein Mentholderivat oder eine Kombination davon, wobei das Mentholderivat die Cyclohexaneinheit umfasst und mit Funktionsgruppen einschließlich Carboxamid, Ketal, Ester, Ether, Alkohol derivatisiert ist; und
wobei das Gewichtsverhältnis von oben erwähnten (a) : (b) von 1 : 60 bzw. 11 : 60 beträgt; und
wobei die Zusammensetzung ferner ein C6-Alkanyldiol; ein C8-Alkanyldiol oder eine Kombination davon umfasst.

2. Zusammensetzung nach Anspruch 1, wobei das Gewichtsverhältnis von (a) : (b) von 2 : 60 bzw. 11 : 60; vorzugsweise von 5 : 60 bis 10 : 60; mehr bevorzugt von 7 : 60 bis 9 : 60 beträgt.

3. Zusammensetzung nach Anspruch 1 oder 2, wobei die Zusammensetzung die Kombination eines C6-Alkanyldiols und eines C8-Alkanyldiols umfasst.

4. Zusammensetzung nach einem der vorstehenden Ansprüche, wobei das Gewichtsverhältnis von (TRPV1-Agonist): (das C6-Alkanyldiol, das C8-Alkanyldiol oder die Kombination davon) von 4 : 1 bzw. 4 : 16; vorzugsweise von 4 : 3 bis 4 : 9 beträgt.

5. Zusammensetzung nach einem der vorstehenden Ansprüche, wobei die Zusammensetzung zu 0,005 Gew.-% bis 1 Gew.-% den TRPV1-Agonisten, bezogen auf das Gewicht der Zusammensetzung; vorzugsweise von zu 0,01 bis 0,08 Gew.-%, mehr bevorzugt von zu 0,02 bis 0,06 Gew.-%, noch mehr bevorzugt von zu 0,03 bis 0,05 Gew.-% den TRPV1-Agonisten, bezogen auf das Gewicht der Zahncremezusammensetzung umfasst.

6. Zusammensetzung nach einem der vorstehenden Ansprüche, wobei die Zusammensetzung von zu 0,001 Gew.-% bis 1 Gew.-% Menthol, Mentholderivat oder eine Kombination davon, bezogen auf das Gewicht der Zusammensetzung; vorzugsweise von zu 0,01 bis 0,7 Gew.-%, mehr bevorzugt von zu 0,1 Gew.-% bis 0,6 Gew.-%, noch mehr bevorzugt größer als 0,2 Gew.-% bis 0, 4 Gew.-% das Menthol, Mentholderivat oder einer Kombination davon, umfasst.

7. Zahncremezusammensetzung nach einem der vorstehenden Ansprüche, wobei das Menthol oder Mentholderivat aus L-Menthol, 1-Menthol, Methon und Kombinationen davon; vorzugsweise 1-Menthol, ausgewählt ist;

8. Zahncremezusammensetzung nach einem der vorstehenden Ansprüche, wobei das C6-Alkanyldiol ein geradkettiges C6-Alkandiol ist;
vorzugsweise wird das geradkettige C6-Alkandiol aus 1,2-Hexandiol, 1,6-Hexandiol oder einer Mischung davon ausgewählt ist;
mehr bevorzugt das geradkettige C6-Alkandiol 1,2-Hexandiol ist.

9. Zahncremezusammensetzung nach einem der vorstehenden Ansprüche, wobei das C8-Alkanyldiol ein geradkettiges C8-Alkandiol ist;
vorzugsweise das geradkettige C8-Alkandiol aus 1,2-Octandiol, 1,8-Octandiol oder einer Mischung davon ausgewählt ist;
mehr bevorzugt ist das geradkettige C8-Alkandiol 1,2-Octandiol ist.

10. Zahncremezusammensetzung nach einem der vorstehenden Ansprüche, wobei das C6-Alkanyldiol und das C8-Alkanyldiol 1,2-Hexandiol bzw. 1,2-Octandiol sind.

11. Zahncremezusammensetzung nach einem der vorstehenden Ansprüche, wobei der TRPV1-Agonist aus der Gruppe ausgewählt ist, bestehend aus Capsaicin, Vanilloiden, Piperin, Dialkdhydsesquiterpen, Triprenylphenol, Ginerolen, Shogaolen und Kombinationen davon;
vorzugsweise wobei der TRPV1-Agonist ein Vanilloid ist;
mehr bevorzugt das Vanilloid aus N-Vanillylalkandienamiden, N-Vanillylalkandienylen, N-Vanillyl-cis-monoungesättigten Alkenamiden, Capsaicin, Dihydrocapsaicin, Norhydrocapsaicin, Nordihydrocapsaicin, Homocapsaicin, Homodihydrocapsaicin und Kombinationen davon ausgewählt ist;
noch mehr bevorzugt das Vanilloid Vanillylbutylether ist.

12. Zahncremezusammensetzung nach einem der vorstehenden Ansprüche, ferner umfassend von zu 0,1 Gew.-% bis 25 Gew.-% ein Poliermittel, bezogen auf das Gewicht der Zusammensetzung; vorzugsweise wobei das Poliermittel Silica ist, mehr bevorzugt die Zusammensetzung von zu 5 bis 25 Gew.-% Silica umfasst.

13. Zahncremezusammensetzung nach einem der vorstehenden Ansprüche, ferner umfassend ein Feuchthaltemittel;
wobei vorzugsweise das Feuchthaltemittel ein Polyol ist;
mehr bevorzugt das Polyol aus Sorbit, Glycerin oder einer Kombination davon ausgewählt ist; noch mehr bevorzugt von zu 1 Gew.-% bis 60 Gew.-% das Feuchthaltemittel, vorzugsweise von zu 40 Gew.-% bis 55 Gew.-%, bezogen auf das Gewicht der Zahncremezusammensetzung vorliegt.

14. Zahncremezusammensetzung nach einem der vorstehenden Ansprüche, umfassend:
(a) Wasser, vorzugsweise zu 5 Gew.-% bis 30 Gew.-% Wasser, mehr bevorzugt von zu 15 Gew.-% bis 25 Gew.-% Wasser, bezogen auf das Gewicht der Zahncremezusammensetzung;
(b) ein Verdickungspolymer, vorzugsweise wobei das Verdickungspolymer von zu 0,1 Gew.-% bis 10 Gew.-% bezogen auf das Gewicht der Zahncremezusammensetzung umfasst;
(c) einen pH-Wert von 5 bis 9, vorzugsweise von 6 bis 8, mehr bevorzugt von 6,5 bis 7,5; und
(d) eine Viskosität von 150.000 10⁻³ Pas (Centipoise) bis 850.000 10⁻³ Pas (Centipoise), gemessen bei Umgebungstemperatur mit einem Brookfield ^{®} Viskosimeter, Modell DV-I Prime mit einem Brookfield "Helipath"-Ständer, wobei das Viskosimeter auf dem Helipath-Ständer platziert und über Wasserwaagen nivelliert wird; die E-Spindel angebracht wird, und das Viskosimeter auf 2,5 U/min eingestellt wird; dann wird die Spindel gelöst, das Viskosimeter auf null gestellt und die E-Spindel installiert und abgesenkt, bis das Querstück teilweise in die Paste eingetaucht ist, bevor mit der Messung begonnen wird; der Netzschalter an dem Viskosimeter an dem Helipath gleichzeitig eingeschaltet wird, um die Abwärtsrotation der Spindel zu beginnen und die Viskosität nach 48 Sekunden bei Raumtemperatur in Centipoise abgelesen wird; und
(e) optional die Zahncremezusammensetzung weniger als zu 0,001 Gew.-% andere Alkanyldiole als das C6-Alkanyldiol oder das C8-Alkanyldiol umfasst und vorzugsweise frei von diesen ist.

15. Zahncremezusammensetzung nach den Ansprüchen 1 bis 14 zur Verwendung zum Bereitstellen von:
(i) einem dominanten Wärmegefühl in der Mundhöhle, umfassend den Schritt des Bürstens mit der Zahncremezusammensetzung für 60 Sekunden bis 120 Sekunden; und
(ii) einem folgenden dominanten Kühlgefühl in der Mundhöhle nach dem Ausstoßen der Zahncremezusammensetzung aus der gebürsteten Mundhöhle.

## Revendications

1. Composition de dentifrice comprenant :
(a) un agoniste de récepteur à potentiel de récepteur transitoire vanilloïde-1 (« TRPV1 ») ;
(b) le menthol, ou un dérivé du menthol, ou une combinaison de ceux-ci, dans laquelle le dérivé du menthol comprend le fragment cyclohexane, et est dérivé avec des groupes fonctionnels, notamment carboxamide, cétal, ester, éther, alcool ; et
dans laquelle le rapport en poids de (a):(b) susmentionné va de 1:60 à 11:60 respectivement ; et
dans laquelle la composition comprend en outre un alcanyl diol en C6 ; un alcanyl diol en C8, ou une combinaison de ceux-ci.

2. Composition selon la revendication 1, dans laquelle ledit rapport en poids dudit (a):(b) va de 2:60 à 11:60, respectivement ; de préférence de 5:60 à 10:60 ; plus préférablement de 7:60 à 9:60.

3. Composition selon la revendication 1 ou 2, dans laquelle la composition comprend la combinaison d'un alcanyl diol en C6 et d'un alcanyl diol en C8.

4. Composition selon l'une quelconque des revendications précédentes, dans laquelle le rapport en poids de (l'agoniste TRPV1) : (ledit alcanyl diol en C6, ledit alcanyl diol en C8 ou ladite combinaison de ceux-ci) va de 4:1 à 4:16, respectivement ; de préférence de 4:3 à 4:9.

5. Composition selon l'une quelconque des revendications précédentes, dans laquelle la composition comprend de 0,005 % à 1 % de l'agoniste TRPV1, en poids de la composition ; de préférence de 0,01 à 0,08 % en poids, plus préférablement de 0,02 à 0,06 % en poids, encore plus préférablement de 0,03 à 0,05 % en poids dudit agoniste TRPV1, en poids de la composition de dentifrice.

6. Composition selon l'une quelconque des revendications précédentes, dans laquelle la composition comprend de 0,001 % à 1 % de menthol, de dérivé de menthol ou d'une combinaison de ceux-ci, en poids de la composition ; de préférence de 0,01 à 0,7 % en poids, plus préférablement de 0,1 % en poids à 0,6 % en poids, encore plus préférablement de plus de 0,2 % en poids à 0,4 % en poids dudit menthol, dudit dérivé de menthol ou de ladite combinaison de ceux-ci.

7. Composition de dentifrice selon l'une quelconque des revendications précédentes, dans laquelle le menthol ou le dérivé de menthol est choisi parmi le L-menthol, le 1-menthol, la méthone et des combinaisons de ceux-ci ; de préférence le 1-menthol.

8. Composition de dentifrice selon l'une quelconque des revendications précédentes, dans laquelle l'alcanyl diol en C6 est un alcane diol à chaîne linéaire en C6 ;
de préférence, l'alcane diol à chaîne linéaire en C6 est choisi parmi le 1,2-hexanediol, le 1,6-hexanediol et des mélanges de ceux-ci ;
plus préférablement, l'alcane diol à chaîne linéaire en C6 est le 1,2-hexanediol.

9. Composition de dentifrice selon l'une quelconque des revendications précédentes, dans laquelle l'alcanyl diol en C8 est un alcane diol à chaîne linéaire en C8 ;
de préférence, l'alcane diol à chaîne linéaire en C8 est choisi parmi le 1,2-octanediol, le 1,8-octanediol et des mélanges de ceux-ci ;
plus préférablement, l'alcane diol à chaîne linéaire en C8 est le 1,2-octanediol.

10. Composition de dentifrice selon l'une quelconque des revendications précédentes, dans laquelle l'alcanyl diol en C6 et l'alcanyl diol en C8 sont respectivement le 1,2-hexanediol et le 1,2-octanediol.

11. Composition de dentifrice selon l'une quelconque des revendications précédentes, dans laquelle l'agoniste TRPV1 est choisi dans le groupe constitué de : capsaïcine, vanilloïdes, pipérine, dialkdhyde sesquiterpène, triprénylphénol, ginérols, shogaols, et combinaisons de ceux-ci ;
de préférence dans laquelle l'agoniste TRPV1 est un vanilloïde ;
plus préférablement, le vanilloïde est choisi parmi les N-vanillyl-alcanediénamides, les N-vanillyl-alcanediényles, les alcénamides N-vanillyl-cis-mono-insaturés, la capsaïcine, la dihydrocapsaïcine, la norhydrocapsaïcine, la nordihydrocapsaïcine, l'homocapsaïcine, l'homodihydrocapsaïcine et des combinaisons de ceux-ci ;
encore plus préférablement, le vanilloïde est l'éther de vanillyle et de butyle.

12. Composition de dentifrice selon l'une quelconque des revendications précédentes, comprenant en outre de 0,1 % à 25 % d'un abrasif, en poids de la composition ; de préférence, dans laquelle l'abrasif est de la silice, plus préférablement, la composition comprend de 5 à 25 % en poids de la silice.

13. Composition de dentifrice selon l'une quelconque des revendications précédentes, comprenant, en outre, un humectant ;
de préférence, l'humectant est un polyol ;
plus préférablement, le polyol est choisi parmi le sorbitol, la glycérine ou une combinaison de ceux-ci ;
encore plus préférablement, de 1 % à 60 % de l'humectant, de préférence de 40 % à 55 %, en poids de la composition de dentifrice.

14. Composition de dentifrice selon l'une quelconque des revendications précédentes, comprenant :
(a) de l'eau, de préférence de 5 % à 30 % d'eau, plus préférablement de 15 % à 25 % d'eau, en poids de la composition de dentifrice ;
(b) un polymère épaississant, de préférence dans laquelle le polymère épaississant représente de 0,1 % à 10 % en poids de la composition de dentifrice ;
(c) a pH allant de 5 à 9, de préférence de 6 à 8, encore plus préférablement de 6,5 à 7,5 ; et
(d) une viscosité allant de 150 000 10⁻³ Pa·s (centipoise) et 850 000 10-³ Pa·s (centipoise), telle que mesurée à température ambiante à l'aide d'un viscosimètre Brookfield ^{®}, modèle DV-I Prime, équipé d'un pied Brookfield "Helipath", dans laquelle le viscosimètre est placé sur le pied Helipath et mis à niveau à l'aide d'un niveau à bulle ; la broche E est fixée et le viscosimètre est réglé à 2,5 tr/min ; puis la broche est détachée, le viscosimètre est mis à zéro et la broche E est installée et abaissée jusqu'à ce que la traverse soit partiellement immergée dans la pâte avant de commencer la mesure ; l'interrupteur d'alimentation du viscosimètre et de l'helipath est mis en marche simultanément pour amorcer la rotation de la broche vers le bas et la viscosité est lue après 48 secondes en centipoise à température ambiante ; et
(e) éventuellement, ladite composition de dentifrice comprend moins de 0,001 % en poids, et de préférence est exempte, d'alcanyl diols autres que ledit alcanyl diol en C6 ou ledit alcanyl diol en C8.

15. Composition de dentifrice selon les revendications 1 à 14, destinée à être utilisée pour fournir :
(i) une sensation de chaleur dominante dans une cavité buccale, comprenant l'étape du brossage avec la composition de dentifrice entre 60 secondes et 120 secondes ; et
(ii) une sensation de refroidissement qui domine ensuite dans la cavité buccale après l'expulsion de ladite composition de dentifrice de la cavité buccale brossée.
